# EUROPEAN PATENT APPLICATION

(11) **EP 1 840 133 A1**
(43) Date of publication of application: **03.10.2007**
(21) Application number: 06075729.1
(22) Date of filing: 29.03.2006
(51) Int. Cl.: C07K 5/08, C07K 7/06, A61K 38/06, A61K 38/08

(54) **New immunomodulating oligopeptides**

(71) Applicant: Global Biotech Development Corp. Ltd., Tortola (VG)
(72) Inventor: Litvinov, Sergey V., 1060 RW Amsterdam (NL)
(74) Representative: van Loon, C.J.J.

(57) **Abstract**

The invention provides immunomodulating peptides of the general formula X-P-Y and/or oligopeptides comprising this amino acid sequence and/or conjugates with these peptides. Also provided are pharmaceutical compounds and compositions with these peptides and conjugates.

## Description

The invention is in the field of immunology, more specifically relating to immunomodulating oligopeptides.

Studies of biologically active short polypeptide sequences (oligopeptides) started from the late 1970s, when the first thymus originating peptides affecting the immune system were discovered.

One such a peptide is the tripeptide Tyr-Gly-Gly (YGG) (also known under the name Imreg; US 4,699,898), which is a weak thymomimetic. Other examples are Tuftsin (also named Tafcin) and its close analogue Rigin, with the sequences Thr-Lys-Pro-Arg (TKPR) and Gly-Gln-Pro-Arg (GQPR), respectively, see for example Biondi et al., Int. J. Peptide Protein Res. 37, 112-121, 1991 and Verdini et al., J. Med. Chem. 34, 3372-3379, 1991. Tuftsin and its analogues are fragments of IgG, e.g. found within the heavy chain of leukokinin. Tuftsin is a well-known macrophage activator and is known to stimulate NK activity. Other fragments are the pentapeptide thymopentin, Arg-Lys-Asp-Val-Tyr (RKDVY) or its tetrapeptide Arg-Lys-Asp-Val (RKDV) and its analogue splenopeptin Arg-Lys-Glu-Val-Tyr (RKEVY) (US 5,091,510; Audhya et al., Proc. Natl. Acad. Sci. USA 81, 2847-2849, .1984). They are thymomimetics of weak potency inducing phenotypic differentiation of T cells and increasing IL-2 production. Form brown marine algae eisenin is isolated, L-pyroGlu-L-Gln-L-Ala (Kojima et al., J. Immunother. 13, 36-42, 1993) which shows immunological activity augmenting natural cytotoxicity of peripheral blood lymphocytes (PBLs) in humans.

Oligopeptides according to the general scheme X-R₁-R₂-R₃-R₄-Y, in which X is selected from the group consisting of H or acetyl; R₁ is selected from the group consisting of D or L-lysine; arginine, ornithine and histidine; R₂ is selected from the group consisting of D or L-asparagine, alanine, praline, glutamine, serine, threonine and valine; R₃ is selected from the group consiting of D- or L-proline; alanine, asparagines, glutamine, serine, threonine, valine and glycine; R₄ is selected from the group consiting of D or L-tyrosine, cysteine, serine, threonine, phenylalanine, tryptophan and histidine; and Y is selected from the group consisting of OH, NH₂ and OC₁₋₆ alkyl, are disclosed in US 5,980,913, where specifically several peptides comprising the sequence (D,L)Lys-Asn/Gln-Pro/Gly-Tyr (K[N/Q][P/G]Y) are mentioned. These peptides have been shown to be useful in the treatment of immune deficiencies.

Further, the dipeptide Glu-Trp (EW, Timogen) and the tripeptide or tetrapeptide X-Glu-Trp-Y, wherein X and Y can be substantially all naturally amino acids, have been described (WO 89/06134; WO 93/08815 and US 6,159,940). From these the tripeptide Ile-Glu-Trp (IEW), also called Neogen, has been used in therapy to treat immune and/or hemopoietic disorders.

Several oligopeptides, among which the above, which are known to have biological function are listed in Table 1. These may be divided into three main groups by their origin:
1. Isolates from thymus, spleen or myeloid tissues, which are seemingly peptides that are employed in the immune system at different stages of immune cell growth and functional differentiation.
2. Products of protein degradation (such as from milk casein, immunoglobulins, etc.) that possess immunomodulating functions, as a rule related to functions of macrophages.
3. Neuromodulating peptides that, through complex interactions between immune and neurological systems, have effect on the immunological reactions of the organism.

Historically, the direction of the development of such compounds was based, and is still largely based, on search and further development of naturally occurring peptides with biological activity. Some attempts of creating short biological peptides were made by several research groups trying to identify the shortest functional motifs in cytokines, adhesion proteins and some surface receptors.

Beyond this, now an attempt was undertaken to perform an extensive screening for oligopeptides demonstrating the required biological properties. We believe that there is still need for alternative peptide immunomodulators.

### SUMMARY OF THE INVENTION

In the course of a screening program the present inventors now have found a new class of immunoactive oligopeptides.

Hence, the invention is related to an oligopeptide, which is a tripeptide of the general formula X-Pro-Tyr (X-P-Y) wherein X can be chosen from the group consisting of Ile (I), Val (V), Ala (A), Trp (W), Pro (P), Leu (L), Phe (F), Gly (G), Glu (E), Asn (N) and Tyr (Y), wherein all amino acids can be in the D or L configuration.

In another embodiment, the invention relates to an oligopeptide comprising at the N-terminus the above mentioned tripeptide.

Specifically, the invention is related to an oligopeptide selected from the group consisting of:
IPYKTTKS, KTTKSIPY, IPYVGVAPG, VGVAPGIPY, IPYVGV, VGVIPY, IPYIEW, IEWIPY, EWIPY, IPYKE, KEIPY, IPYKPR, KPRIPY, IPYTEPR, TEPRIPY, IPYKD, KDIPY, IPYKNPY, KNPYIPY, IPYKNPW, KNPWIPY, IPYTKPR, IPYGQPR, IPYTAEEK, IPYALTTE, IPYRKEVY, GPAIPY, KDIPIPY, TQPIPY, GQPIPY, TAEIPY, ALTIPY, RKEIPY, IPYEKX₁ EKX₁IPY, IPYX₁EK X₁EKIPY, IPYEWX₁, EWX₁IPY IPYX₁VY, X₁VYIPY, IPYX₁PY, X₁PYIPY, IPYPRX₁, and PRX₁IPY wherein X₁ is selected from the group consisting of A, V, G, I, P, W, F, N, Q, T, R, K, E, D and Y.
In another embodiment the above mentioned oligopeptides are conjugated to palmitic acid.
The above mentioned oligopeptides according to the invention or a conjugate according to the invention are useful in therapy, preferably in activating T-cells and/or inducing release of cytokines and/or increasing T-cell dependent humoral and/or cellular immune response and/or inhibiting autoimmune responses.

Another embodiment is a pharmaceutical compound comprising an oligopeptide or a conjugate according to the invention and a pharmaceutically acceptable carrier. This pharmaceutical compound can be used for the samen therapeutic purposes as mentioned above.

Alternatively, this invention comprises the use of an oligopeptide or a conjugate according to the invention for the manufacture of a medicament for use as immunomodulator, preferably for use in activating T-cells and/or inducing release of cytokines and/or increasing T-cell dependent humoral and/or cellular immune response and/or inhibiting autoimmune responses. Also a vaccine comprising an oligopeptide or a conjugate of the invention is comprised in the invention.

In another embodiment, the invention comprises a method to confer immunoreactivity to a peptide or to enhance immunoreactivity of a peptide by fusing said peptide with a peptide of the invention

### DESCRIPTION OF THE FIGURES

Fig. 1 shows the effect of B134 (tripeptide Ile-Pro-Tyr; dark gray line) and B211 (oligopeptide IEWIPY, light grey line) on immunoglobulin concentrations (of respectively IgG1, IgG2a, IgG2b and IgM) in mouse serum after immunisation vs. control (black line).

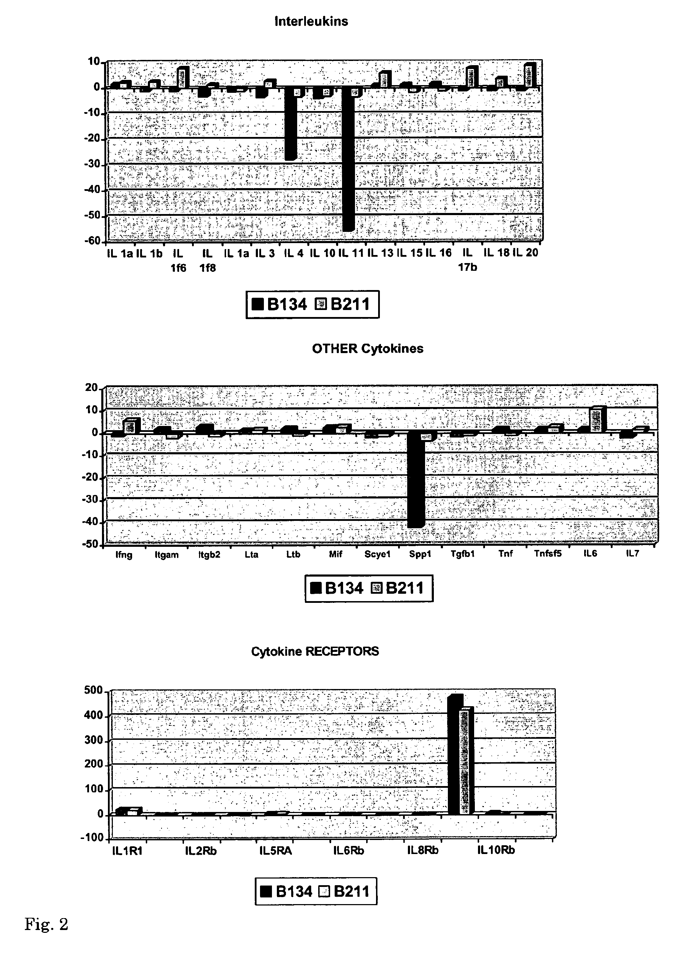

Fig. 2 shows changes in the expression of immune response related genes (interleukins, cytokines and cytokine receptors) as a result of co-vaccination with either B134 (dark grey bars) or B211 (light grey bars).
Control expression set at 0.

### DETAILED DESCRIPTION OF THE INVENTION

An oligopeptide according to the present invention is a chemical compound consisting of amino acids, connected trough peptide links with a maximal length of about 50 amino acids (about 1-10 kDalton). An oligopeptide can consist of naturally occurring amino acids (see list of Table 2) or non-naturally occurring amino acids, such as norleucine, ornithine, norvaline, statine , desmosine, GABA , sarcosine, isodesmosine, allo-isoleucine, beta-alanine and derivatives of these, such as 2-aminoadipic acid, 3-aminoadipic acid, 2-aminobutyric acid, piperidinic acid, 6-aminocaproic acid, 2-aminoheptanoic acid, 2-aminoisobutyric acid, 3-aminoisobutyric acid, 2-aminopimelic acid, 2,4-diamonobutyric acid, 2,2'-diaminopimelic acid, 2,3-diaminopropionic acid, N-ethylgl;ycine, N-ethylasparagine, all-hydroxylysine, hydroxylysine, 3-hydroxyproline, 4-hydroxyproline, N-methylisoleucine, 6-N-methyllysine, N-methylvaline and others (see for further examples e.g. Hunt, S., The non-protein amino acids. In: Chemistry and Biochemistry of the Amino Acids. Barrett, G.C. (ed.), Chapman and Hall, London, 1985).. Further, an oligopeptide can have all L amino acids, or all D amino acids or combinations thereof.

In this application, peptides are designated by their three letter code or by their one-letter IUPAC code according to the following Table:

**Table 2. list of naturally occurring amino acids**

| | | |
|---|---|---|
| alanine | Ala | A |
| arginine | Arg | R |
| asparagines | Asn | N |
| aspartic acid | Asp | D |
| cysteine | Cys | C |
| glutamine | Gln | Q |
| glutamic acid | Glu | E |
| glycine | Gly | G |
| histidine | His | H |
| isoleucine | Ile | I |
| leucine | Leu | L |
| lysine | Lys | K |
| methionine | Met | M |
| phenylalanine | Phe | F |
| praline | Pro | P |
| serine | Ser | S |
| threonine | Thr | T |
| tryptophan | Trp | W |
| tyrosine | Tyr | Y |
| valine | Val | V |

When D- or L-isostereomeres are indicated these are specifically meant, if no indication is given the aminoacid can be either an L- or D-isomeres

It was found that the tripeptides having the general formula X-Pro-Tyr (X-P-Y) wherein X can be chosen from the group consisting of Ile (I), Val (V), Ala (A), Trp (W), Pro (P), Leu (L), Phe (F), Gly (G), Glu (E), Asn (N) and Tyr (Y), and wherein all amino acids can individually be in the D or L configuration, have immunomodulating properties. Preferably, the tripeptide is IPY, which has been tested extensively (see experimental part).

Surprisingly it has also been found that this tripeptide maintains its immunomodulating activity when joined to another peptide, whether this peptide also has immunomodulating activity or not. If it has immunostimulating activity (such as the peptide IEW) the hexapeptides IEWIPY or IPYIEW will show an enhanced immunoactivity with respect to the parent compound IEW. It has not appeared to make much difference whether the IPY tripeptide is situated at the N-terminal or at the C-terminal end of the peptide to which it is joined. However, a preferred group is the group of peptides which have IPY at their N-terminus. It is even complicated in the present invention, that the tripeptide does not need to be situated at the termini of the peptide with which it is joined, it would also be possible to engineer the tripeptide within in a peptide sequence, with the proviso that it is situated at a position in which it can be exposed to the environment and thus exert its function. Also, of course, introduction of the tripeptide in another peptide should leave the original function of the other peptide intact, if desired.

When joined to a peptide antigen or epitope, it will enhance the immunesystem to start proliferating antibodies to said antigen or epitope. Thus, the tripeptide is an ideal adjuvans for boosting immunresponses to a vaccination, and for ease of administration and stability, the tripeptide may be joined to the antigen/epitope if that is proteinaceous.

Specific examples of oligopeptides which comprise the tripeptide of the invention are IPYKTTKS, KTTKSIPY, IPYVGVAPG, VGVAPGIPY, IPYVGV, VGVIPY, IPYIEW, IEWIPY, EWIPY, IPYKE, KEIPY, IPYKPR, KPRIPY, IPYTEPR, TEPRIPY, IPYKD, KDIPY, IPYKNPY, KNPYIPY, IPYKNPW, KNPWIPY, IPYTKPR, IPYGQPR, IPYTAEEK, IPYALTTE, IPYRKEVY, GPAIPY, KDIPIPY, TQPIPY, GQPIPY, TAEIPY, ALTIPY, RKEIPY, IPYEKX₁ EKX₁IPY, IPYX₁EK X₁EKIPY, IPYEWX₁, EWX₁IPY IPYX₁VY, X₁VYIPY, IPYX₁PY, X₁PYIPY, IPYPRX₁, and PRX₁IPY wherein X₁ is selected from the group consisting of A, V, G, I, P, W, F, N, Q, T, R, K, E, D and Y. As the skilled person will notice, many of the listed oligomers (predominantly hexapeptides) occur in the list of Table 1, i.e. they are known oligopeptides which have proven immunological effects. Although the list above only mentions combinations with IPY, in general, all combinations wherein the IPY is replaced by the tripeptides of the invention, i.e. those having the general formula X-Pro-Tyr (X-P-Y) wherein X can be chosen from the group consisting Val (V), Ala (A), Trp (W), Pro (P), Leu (L), Phe (F), Gly (G), Glu (E), Asn (N) and Tyr (Y), and wherein all amino acids can individually be in the D or L configuration, have immunomodulating properties. In the case of these combination peptides, the resulting peptide does not need to be an oligopeptide. It is envisaged, especially in cases where the tripeptide is joined to an antigen, that the resulting peptide can be of any length.

The peptides or peptide derivatives of the invention can be produced synthetically or, where applicable, recombinantly by conventional methods. Specific embodiments of the oligopeptides are disclosed in detail in the experimental part below. Preferably, the oligopeptides of the invention are prepared conventionally by known chemical synthesis techniques, such as, for instance, are disclosed by Merrifield (J. Am. Chem. Soc. (1963) 85:2149-2154).

Alternatively, the (oligo)peptides of the invention may be produced by recombinant DNA techniques by cloning and expressing within a host micro-organism or cell a DNA fragment carrying a nucleic acid sequence encoding one of the above-described peptides. Nucleic acid coding sequences can be prepared synthetically, or may be derived from existing nucleic acid sequences by site-directed mutagenesis. These nucleic acid sequences may then be cloned in a suitable expression vector and transformed or transfected into a suitable host cell, such as *E. coli, Bacillus, Lactobacillus, Streptomyces,* mammalian cells (such as CHO, HEK or COS-1 cells), yeasts (e.g. *Saccharomyces, Schizophyllum),* insect cells or viral expression systems, such as baculovirus systems. A person skilled in the art will have knowledge of the techniques of constructing the nucleic acid sequences and providing means to enable their expression.

It is also possible to include non naturally occurring amino acids (like D-amino acids) in peptides through genetic engineering techniques. This has been extensively described in Noren et al., Science 244:182 (1989) and Ellman et al. Meth. Enzymol. 202:301 (1991).

Subsequently, the peptide can be isolated from the culture of the host cells. This can be achieved by common protein purification and isolation techniques which are available in the art. Such techniques may e.g. involve immunoadsorption or chromatography. It is also possible to provide the peptides with a tag (such as a histidine tag) during synthesis, which allows for a rapid binding and purification, after which the tag is enzymatically removed to obtain the active peptide.

If the peptide itself cannot be encoded or expressed but is very similar to a peptide that can be encoded or expressed, the method can be applied to prepare the peptide to which the peptide is similar, followed by one or more steps in which said peptide is modified by chemical or enzymatic techniques to prepare the final peptide.

The oligopeptides can also be obtained by cleaving the oligopeptide off from a larger peptide using proteolytic enzymes, like pepsine, papaine, etc.

Some more comprehensive summaries of methods which can be applied in the preparation of the peptides are described in: W. F. Anderson, Nature 392 Supp., 30 April 1998, p. 25-30; Pharmaceutical Biotechnology, Ed. D. J. A. Crommelin and R. D. Sindelar, Harwood Academic Publishers, 1997, p. 53-70, 167-180, 123-152, 8-20; Protein Synthesis: Methods and Protocols, Ed. R. Martin, Humana Press, 1998, p. 1-442; Solid-Phase Peptide Synthesis, Ed. G. B. Fields, Academic Press, 1997, p. 1-780; Amino Acid and Peptide Synthesis, Oxford University Press, 1997, p. 1-89.

Novel peptides according any of claims 1-3 can be readily made by a person skilled in the art.

A special embodiment of the current invention is formed by conjugates of any of the oligopeptides of the invention to palmatic acid. Such conjugation allows better penetration of the peptides of the invention through the skin and other epithelial tissues.

A pharmaceutical composition of the invention comprises a therapeutically effective amount of one or more oligopeptides or conjugates of the present invention. Once formulated, the pharmaceutical compositions of the invention can be administered directly to the subject in need thereof in a therapeutically effective amount.

Direct delivery of the compositions will generally be accomplished by topical application or other forms of administration, either orally, parenterally, subcutaneously, sublingually, intranasally, intralesionally, intraperitoneally, intravenously or intramuscularly, pulmonarily, or delivered to the interstitial space of a tissue.

The pharmaceutical composition may also comprise a suitable pharmaceutically acceptable carrier or diluent and may be in the form of a capsule, tablet, lozenge, dragee, pill, droplet, suppository, powder, spray, vaccine, ointment, paste, cream, inhalant, patch, aerosol, and the like. As pharmaceutically acceptable carrier, any solvent, diluent or other liquid vehicle, dispersion or suspension aid, surface active agent, isotonic agent, thickening or emulsifying agent, preservative, encapsulating agent, solid binder or lubricant can be used which is most suited for a particular dosage form and which is compatible with the peptide or peptide conjugate.

A pharmaceutical composition may thus contain a pharmaceutically acceptable carrier. The term "pharmaceutically acceptable carrier" also includes a carrier for administration of a therapeutic agent, such as antibodies or a polypeptide, genes, and other therapeutic agents. The term refers to any pharmaceutical carrier that does not itself has any immunological effect, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Salts of oligopeptides or conjugates are prepared by known methods, which typically involve the mixing of the peptide or conjugate with either a pharmaceutically acceptable acid to form an acid addition salt, or with a pharmaceutically acceptable base to form a base addition salt. Whether an acid or a base is pharmaceutically acceptable can be easily decided by a person skilled in the art after taking the specific intended use of the compound into consideration. For instance, not all acids and bases that are acceptable for *ex vivo* applications can be used for therapeutic compositions, and not all acids and bases which are suitable for topical use can be applied parenterally. Depending on the intended use, pharmaceutically acceptable acids include organic and inorganic acids such as formic acid, acetic acid, propionic acid, lactic acid, glycolic acid, oxalic acid, pyruvic acid, succinic acid, maleic acid, malonic acid, cinnamic acid, sulfuric acid, hydrochloric acid, hydrobromic acid, nitric acid, perchloric acid, phosphoric acid, and thiocyanic acid, which form ammonium salts with free amino groups of peptides and conjugates. Pharmaceutically acceptable bases, which form carboxylate salts with free carboxylic groups of peptides and functional equivalents, include ethylamine, methylamine, dimethylamine, triethylamine, isopropylamine, diisopropylamine, and other mono-, di- and trialkylamines, as well as arylamines. Moreover, also pharmaceutically acceptable solvates are encompassed.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic parenteral compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

For therapeutic treatment, oligopeptide or conjugate may be produced as described above and applied to the subject in need thereof. The peptide or peptide-conjugate may be administered to a subject by any suitable route, preferably in the form of a pharmaceutical composition adapted to such a route and in a dosage which is effective for the intended treatment.

Pharmaceutical compositions of this invention may contain other active agents, such as antigens or epitopes (like e.g. used in common vaccines) or other immunologically active compounds, such as any peptide from Table 1. Also combinations with anti-viral agents (such as AZT), anti-anaemic drugs (like GM-CSF EPO) are contemplated within the invention. Most preferably, the peptides of the invention are co-administered with a vaccine.

Therapeutically effective dosages of the peptide or peptide-conjugate required for evoking an immunomodulating reaction in the body of a human or animal subject, can easily be determined by the skilled person, for instance by using animal models.
The term "therapeutically effective amount" as used herein refers to an amount of a therapeutic, *viz*. an oligopeptide or peptide-conjugate according to the present invention, to show an immunomodulating reaction. A person skilled in the art will be able to determine the amounts of peptide needed to show an immunomodulatory action by determining the humoral immune response, or cell activation of activation of various genes, as is demonstrated in the present examples. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician or experimenter. Specifically, the compositions of the present invention can be used for enhancing the immune reaction by activating T-cells and/or inducing release of cytokines and/or increasing T-cell dependent humoral and/or cellular immune response and/or decreasing the immune reaction, e.g. by inhibiting autoimmune responses. Methods that permit the clinician to establish initial dosages are known in the art. The dosages determined to be administered must be safe and efficacious.

For purposes of the present invention, an effective dose will be from about 0.01 µg/kg to 50 mg/kg, preferably 0.5 µg/kg to about 10 mg/kg of the oligopeptide or peptide-conjugate in the individual to which it is administered. Dosages for achieving the therapeutic effects of the pharmaceutical composition described herein may easily be determined by the skilled person.

Yet in another alternative embodiment, the oligopeptide or peptide-conjugate or compositions of the invention may be administered from a controlled or sustained release matrix inserted in the body of the subject.

It may also be advantageous to administer a compound of the invention in a transmucosal dosage form. This route of administration is non-invasive and patient-friendly; and, especially in the treatment of wounds and sores, it may lead to an improved bioavailability of the compound compared to oral administration, especially if the compound is not stable in the fluids of the digestive system, or if it is too large to be absorbed from the gut effectively. Transmucosal administration is possible, for instance, via nasal, buccal, sublingual, gingival, or vaginal dosage forms, but especially envisaged in this application is the administration to open wounds to ameliorate the wound-healing effect. These dosage forms can be prepared by known techniques; they can be formulated to represent nasal drops or sprays, inserts, films, patches, gels, ointments, or tablets. Preferably, the excipients used for a transmucosal dosage form include one or more substances providing for mucoadhesion, thus prolonging the contact time of the dosage form with the site of absorption and thereby potentially increasing the extent of absorption.

In a further embodiment, the compounds are administered via the pulmonary route, using a metered dose inhaler, a nebulizer, an aerosol spray, or a dry powder inhaler. These kind of dose administration forms are excellently applicable in case of vaccination. Appropriate formulations can be prepared by known methods and techniques. Transdermal, rectal, or ocular administration may also be feasible in some cases.

It can be advantageous to use advanced drug delivery or targeting methods to deliver a compound of the invention more effectively. For instance, if a non-parenteral route of administration is chosen, an appropriate dosage form may contain a bioavailability enhancing agent, which may be any substance or mixture of substances which increases the availability of the compound. This may be achieved, for instance, by the protection of the compound from degradation, such as by an enzyme inhibitor or an antioxidant. More preferably, the enhancing agent increases the bioavailability of the compound by increasing the permeability of the absorption barrier, which is typically a mucosa. Permeation enhancers can act via various mechanisms; some increase the fluidity of mucosal membranes, while others open or widen the gap junctions between mucosal cells. Still others reduce the viscosity of the mucus covering the mucosal cell layer. Among the preferred bioavailability enhancers are amphiphilic substances such as cholic acid derivatives, phospholipids, ethanol, fatty acids, oleic acid, fatty acid derivatives, EDTA, carbomers, polycarbophil, and chitosan.

It was found, as shown in the Examples, that the oligopeptides of the present invention have strong immunomodulating activities related to the activation of the immune system by increasing the level of humoral immune response via activation of genes related to the normal course of immune response to T-helper cell dependent antigens.

This thus means that the oligopeptides of the invention would be applicable in the activation of T cells, thereby inducing release of cytokinins, thereby affecting the activity of other immunological and non-immunological cell populations. In particular it was found that the oligopeptides of the invention can stimulate proliferation, differentiation and activation of immunological cell populations.

One of the applications where this can be used therapeutically, is the effect on T cells residing in epithelial tissues, where these T cells affect processes as growth of epithelial cells, wound healing and hair growth. Another application is to use the effect on T cells to boost humoral and cellular immune responses to external antigens. This means that the oligopeptides can be used as an adjuvant in vaccines for increasing the immune response. Thus, the peptides of the invention can be used in all bacterial and/or viral infections. This use can be therapeutic, but also prophylactic administration of the peptides of the invention is envisaged. In the latter case, a general increase of the immunological resistance will be effectuated.

Further, the oligopeptides of the invention can play a role in the therapy of autoimmune diseases.

The invention will now be illustrated with the following, nonlimiting examples.

### EXAMPLES

### Example 1. Effect of B134 and B211 on humoral immune response to T-cell dependent antigen in mice.

The peptide Ile-Pro-Tyr (IPY, B134) and IEWIPY (B211) are presented as examples of biological activity for a family of peptides covered by current application.
To investigate the effect of B134, a representative of the XPY family, inbred SPF mice *(Balb*/*cAwNCrl,* 7 to 8 week old, Charles River Laboratories GmbH, Germany) were immunized with KLH, a T-cell dependent antigen. 3 mice per group were injected subcutaneously in the presence of complete Freund adjuvant (50/50 v/v). The mix of antigen (20 µg in 100 µl) with adjuvant (Sigma, #F-5881) was emulsified and injected in the neck region. At the same day 20 µg of immunomodulator B134 or B211 were injected in 200 µl of PBS intraperitoneally. Samples of blood (50-70 µl) were collected from mice on day 7, 14, 21, and 28 from the leg vein. Serum was prepared by clotting the blood for 2 hours at 37°C, followed by 18 hours at 8°C, and centrifugation at 10,000 rpm in an Eppendorf-like centrifuge. Sera were stored diluted with antibody stabilizer (SkyTec ABB500) at 4°C, and analyzed in ELISA simultaneously. For the latter assay, the 96 wells ELISA plates (Greiner, #655061) were coated with KLH (soluble, Sigma H7017) in phosphate buffered solution (PBS), 0.2 µg/well overnight at 4°C. The diluted sera were incubated with antigen (200 µg/well) for 1 hour at room temperature, followed by a wash with PBS/0.1% Tween-20. The binding of mouse antibodies to KLH was detected using isotype specific anti-mouse immunoglobulins, conjugated with HRP (Southern Biotechnology Ltd., anti-mouse IgM #1021-05, anti-mouse IgG1 #1070-05, anti-mouse IgG2a # 1080-05, anti-mouse IgG2b #1090-05) according to the manufacturer's protocol. TMB was used as a substrate. The results were analyzed on Bio-Rad Microplate Reader Model 550, the optical density was measured at 595 nm.

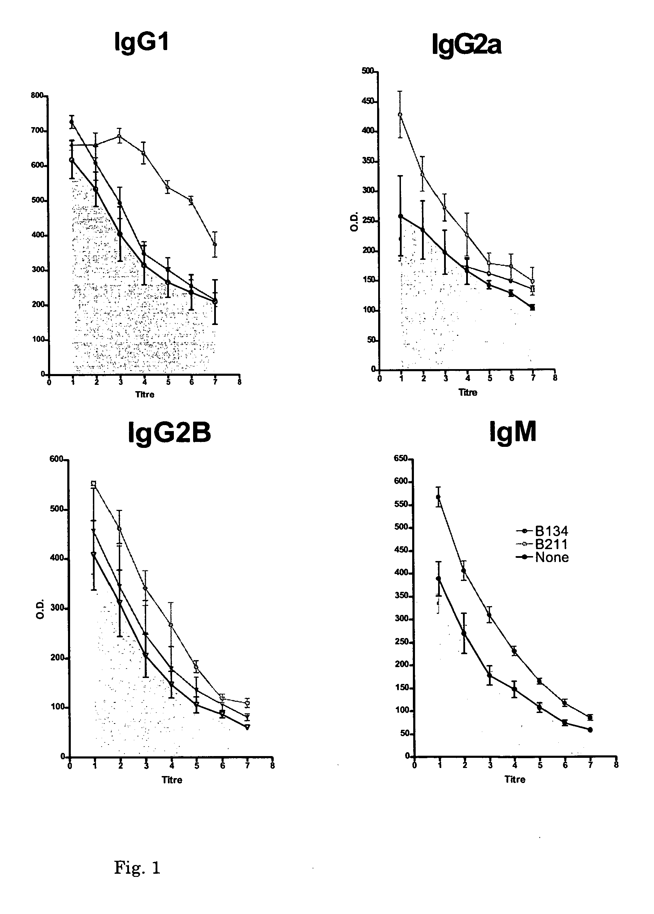

Results shown in Fig. 1 represent titration slope of the serum from experimental mice. The dilutions of sera used are from 1/300 to 1/20,000 with a step of ½ (indicated on X axis as 1 to 6 respectively). The serum reactivity is presented as O.D. shown by a sample in ELISA. The dots represent the average reactivity of samples from 3 sera (from 3 mice per condition). The variation bar represents the 95% confidence interval.
As can be seen from Fig. 1, after a single injection the specific antibody titer on day 28 differs substantially between mice immunized with and without immunomodulator. Thus, the titre of specific IgG1 in sera from mice immunized in the presence of B211 was approximately 16 times higher, and the titer of IgG2a and IgG2b 4 times higher then in control mice, immunized with antigen alone. For B134 the difference was approximately 2 times higher for every isotype analyzed.

### Example 2. Effect of B134 on gene expression in mouse splenocytes as determined by PCR array.

Inbred SPF Balb/c mice (females, 12 weeks old) were injected with either antigen or peptide or a combination of both. For injections, 25 µg of the peptide was solved in 250 µl of sterile PBS. Injections were performed subcutaneously, in the neck region, with an insulin needle. Control mice were injected with PBS only.
For antigen injection: a 250 µl suspension of sterile Sheep Red Blood cells (SRBC from Quad Five inc., Cat # 643-100) was injected intraperitoneally via the flank. The Suspension was prepared as 2 ml of the original suspension washed 2 times (1500 rpm, 5 min) with PBS and re-suspended in 2 ml. 10 µl of 50% suspension was diluted in 250 µl of PBS and injected.
48 hours later mice were sacrificed, their spleens isolated and submerged into RNALater (Ambion Inc., Cat # 7021) immediately upon isolation
Samples in RNALater were immediately frozen at -70°C and kept at that temperature until RNA isolation. RNA isolation and PCR array analysis was performed as a service by SuperArray Inc according to their established protocol (www.superarray.com).

RESULTS. The changes in mRNA expression, as based on PCR data, were found to be statistically significant if the difference with the control level of expression were over 3 fold (either increase or decrease). From expression analysis for 84 genes, the level of 75 to 85% of genes in all samples was not statistically different from the control sample (spleen of the mouse injected with PBS instead of both antigen and immunomodulator (not shown). A clear, statistically significant difference was observed for a number of cytokine and chemokine genes, and the respective receptors IL-4, IL-11, Spp1, IL-10RA and to a lesser extent IL-1f6, IL-13, IL-17b, IL-20, IL-6 and IL-1R1).

**Table 1. Known Peptides**

| Peptide | Name | Reference | Mer | Origin | Effects | Possible targets |
|---|---|---|---|---|---|---|
| | | | | | | |
| EW | Timogen | | 2 | Natural isolate from thymus | Stimulation of immune response | Unknown, T-cells, Il-2 production |
| IEW | Neogen | | 3 | Synthetic by extending the EW | Stimulation of immune response, rebuilding of immune system | T-cell precursors, other cells precursors? |
| KEVVEEAEN | | Ciardelli et al., 1982 | 9 | 20-28 of thymosin alpha 1 | Identical to Thymosin a1 | |
| ac- SDKP | | Grillion et al., 1990 | 4 | N-termini of thymosing beta4 | Proliferation activation of T-cells | |
| RKDVY | Thimopeptin | Goldstein et al., 1979; Nash et al, 1981 | 5 | 32-36 of thymopoeitin | Similar to whole tymopoietin, | Induces differentiation of T-lymphocytes in nude mice, activation of NK cells |
| RKEVY | Splenopentin | Audhya et al., 1984 | 5 | 32-36 of bovine thymopoietin 3, isolated from spleen | Does not activate differentiation of T-cells, but activates differentiation of B-cells | B-cells |
| RKDV | | Denes et al. 1986 | 4 | Fragment of thymopeptin | Similar to thimopeptin, increase of IgM producing cells, allograft rejection | |
| RKD | | Denes et al. 1986 | 3 | Ibid | Ibid | |
| R-a-DKVYR | Timohexin | | | | | CFC in bone marrow, IL-4 production |
| KKEVY | | Biswas et al.,1997 | 5 | Synthetic analogues of SP | Stimulate IL-2 production and CD2R expression | |
| D-KKEVY | | Biswas et al.,1997 | 5 | Ibid | Ibid | |
| KSQE | | Abiko, Sekino, 1982 | 4 | Fragment of thimulin | | |
| | | | | | | |
| LVVYPW | Myelopeptides | Petrov et al., 1984-1997 | 6 | Isolate of peptides produced by bone marrow cells | Increase of T-cell response to mitogen | |
| FLGFPT | Myelopeptides | Michailova et al, 1995 | 6 | Isolate of peptides produced by bone marrow cells | | |
| MLTAEEKR | Myelopeptides | | 8 | Isolate of peptides produced by bone marrow cells | | |
| TAEEK | Myelopeptides | Michaltsov et al, 1997 | 5 | Isolate of peptides produced by bone marrow cells | Stimulation of haemopoiesis | |
| VGGEAL | Myelopeptides | | 6 | Isolate of peptides produced by bone marrow cells | | |
| ac-n-SDKP | Goralatid | Masse et al., 1998; Gaudron 1999 | 4 | ? | Stimulation of haemopoiesis, decrease of cytotoxic effects of cytostatics | Inhibits proliferation of bone marrow cells in vitro. In vivo, however, stimulation of CFC of spleen |
| EEDCK | | Cuthbertson et al, 1997; Paukovits et al., 1998 | 5 | | Stimulation of haemopoiesis | Inhibits proliferation of bone marrow cells in vitro. In vivo, however, stimulation of CFC of spleen |
| KKGE | MPF, HUMAN | Owen et al, 1997,1998 | 4 | Neuropeptide, natural | Stimulates neuron growth, proliferation of T-cells | |
| KKGQ | MPF, RAT | | 4 | | Stimulates neuron growth, proliferation of T-cells | |
| DYMGWMDF | Holeocystokin | Molchanova et al., 1992 | 8 | Natural hormone | Stimulates thymus- dependent, but not independent response | Expression of Thy antigen, growth and differentiation of precursors |
| DYMG | | | 4 | Fragment 1 | ? | |
| WMDF | | | 4 | Fragment 2 | ? | |
| FFGLM | | Siemion et al., 1990 | 5 | C-terminus Substance P (7-11) | Suppresses humoral immune response in doses 1-100 mcg/mouse | |
| RPKP | | | 4 | N-terminus of substance P | 0,1 mcg/ml -suppressive, 1-5 mcg/ml-stimulating | |
| GPRP | | | | Analogue of N- of substance | Suppressive | |
| GAAVLEDSQ | Fragment MCF | | 9 | N-terminus of MCF | Stimulates cytotoxic activity of monocytes | |
| LEDSQ | Fragment MCF | | 5 | | Ibid | |
| GAAVL | Fragment MCF | | 5 | | No activity | |
| GAAVLENSQ | Fragment MCF with replacement | | 9 | | No activity | |
| ITGSE | Fragment IL-1 | Siemion et al., 1998 | 5 | | Suppression of immune response, production of IL2 etc. | |
| VQGEESNDK | Fragment IL-1 | Bajpai et al, 1998 | 9 | Fragment of IL 1a, 163-171 | Immunostimulating activity | |
| TKPR | Tafcin | Fridkin, Najjar, 1998 | 4 | Product of Fc degradation | Stimulates phagocyte activity of leucocytes and macrophages, production of IL 1 by mononuclear cells of spleen | |
| TEPR | Synthetic variant of tafcin | Chipens et al., 1990 | 4 | | Decrease of macrophages activity, increase in infections | |
| KPR | Synthetic variant of tafcin | | 3 | Shortened version of Tafcin | Decrease of macrophages activity, increase in infections | |
| TKP | Variant of tafcin from IgE degradation | Auriault et al., 1985 | 3 | IgE degradation product | Inhibition of macrophage activity, other reports-stimulation. May be related to models and doses. | |
| GQPR | Rigin | | 4 | Fragment of IgG CH2 | Stimulates phagocytic activity | |
| RGDS | Domain of fibronectin | | 4 | Binding domain of fibronectin to cells | Stimulates phagocytosis | |
| GP | Fragments of collagen | Mundy et al., 1981 | 2 | Fragments of collagen isolated from urine | Stimulates chemotaxis of peripheral blood monocytes | |
| GPA | Fragments of collagen | | 3 | | Stimulates chemotaxis of peripheral blood monocytes | |
| VEPIPY | | Parker et al., 1984 | 6 | 54-59 of casein | Stimulates in vitro phagocytosis, in vivo protects against K. pheumoniae | |
| KE | Vilon | | 2 | | Stimulate expression of T-cell receptors | |
| gamma-EW | Bestim | | 2 | Analogue of Timogen | Stimulation of T-cell precursors | |
| KNPY | Gagnon's peptide | | 4 | | Stimulation of B-cells | |
| ALTTE | Ogawa's peptide | | 5 | | Stimulation of B-cells | |

## Claims

1. Oligopeptide, which is a tripeptide of the general formula X-Pro-Tyr (X-P-Y) wherein X can be chosen from the group consisting of Ile (I), Val (V), Ala (A), Trp (W), Pro (P), Leu (L), Phe (F), Gly (G), Glu (E), Asn (N) and Tyr (Y), wherein all amino acids can individually be in the D or L configuration.

2. Oligopeptide comprising at the N-terminus the tripeptide of claim 1.

3. Oligopeptide selected from the group consisting of:
IPYKTTKS, KTTKSIPY, IPYVGVAPG, VGVAPGIPY, IPYVGV, VGVIPY, IPYIEW, IEWIPY, EWIPY, IPYKE, KEIPY, IPYKPR, KPRIPY, IPYTEPR, TEPRIPY, IPYKD, KDIPY, IPYKNPY, KNPYIPY, IPYKNPW, KNPWIPY, IPYTKPR, IPYGQPR, IPYTAEEK, IPYALTTE, IPYRKEVY, GPAIPY, KDIPIPY, TQPIPY, GQPIPY, TAEIPY, ALTIPY, RKEIPY, IPYEKX₁ EKX₁IPY, IPYX₁EK X₁EKIPY, IPYEWX₁, EWX₁IPY IPYX₁VY, X₁VYIPY, IPYX₁PY, X₁PYIPY, IPYPRX₁, and PRX₁IPY wherein X₁ is selected from the group consisting of A, V, G, I, P, W, F, N, Q, T, R, K, E, D and Y.

4. Conjugate of an oligopeptide according to any of claims 1-3 with palmitic acid.

5. Oligopeptide according to any of claims 1-3 or a conjugate according to claim 4 for use in therapy.

6. Oligopeptide according to any of claims 1-3 or a conjugate according to claim 4 for use as immunomodulator, preferably for use in activating T-cells and/or inducing release of cytokines and/or increasing T-cell dependent humoral and/or cellular immune response and/or inhibiting autoimmune responses.

7. Pharmaceutical compound comprising an oligopeptide according to any of claims 1 to 3 or a conjugate according to claim 4 and a pharmaceutically acceptable carrier.

8. Pharmaceutical compound according to claim 7 for use as immunomodulator, preferably for use in activating T-cells and/or inducing release of cytokines and/or increasing T-cell dependent humoral and/or cellular immune response and/or inhibiting autoimmune responses.

9. Use of an oligopeptide according any of claims 1-3 or a conjugate according to claim 4 for the manufacture of a medicament for use as immunomodulator, preferably for use in activating T-cells and/or inducing release of cytokines and/or increasing T-cell dependent humoral and/or cellular immune response and/or inhibiting autoimmune responses.

10. Use according to claim 9 in a vaccine formulation.

11. Vaccine comprising an oligopeptide according to any of claims 1 to 3 or a conjugate according to claim 4.

12. Method to confer immunoreactivity to a peptide or to enhance immunoreactivity of a peptide by fusing said peptide with an oligopeptide according to claim 1.
